# EUROPÄISCHE PATENTSCHRIFT

(11) **EP 2 163 893 B1**
(45) Veröffentlichungstag und Bekanntmachung des Hinweises auf die Patenterteilung: **23.01.2013**
(21) Anmeldenummer: 09173187.7
(22) Anmeldetag: 16.05.2007
(51) Int. Cl.: G01N 33/487, G01N 33/49, G01N 1/28, B01L 3/00, G01N 1/18, G01N 33/52

(54) **Separator**
Separator
Séparateur

(30) Priorität: 16.05.2006 DE 202006007817 U
(43) Veröffentlichungstag der Anmeldung: 17.03.2010
(62) Teilanmeldung aus: 07725305.2
(73) Patentinhaber: Pfaff, Dieter, 86633 Neuburg an der Donau (DE)
(72) Erfinder: Pfaff, Dieter, 86633 Neuburg an der Donau (DE)
(74) Vertreter: Klunker . Schmitt-Nilson . Hirsch

(56) Entgegenhaltungen:
- EP-A- 1 477 804
- EP-B1- 0 278 677
- WO-A-93/03673
- DE-A1- 3 130 749
- DE-U1-202006 007 817

## Beschreibung

Die Erfindung betrifft einen Separator und einen Separationsstreifen zum Separieren von Blutserum oder Blutplasma aus einer Blutprobe.

Aus EP 1 477 804 A ist ein SeparationssVeifen zum Abscheiden von Blutserum aus einer Blutprobe bekannt. Dabei ist es notwendig, das Blutserum aus dem Separationsstreifen hinaus zu drücken.

Dies kann beispielsweise dadurch erfolgen, dass mit einem Werkzeug oder Finger, insbesondere einem Fingernagel, auf den Separationsstreifen gedrückt wird und das Werkzeug bzw. der Finger in Richtung einer an einem Ende des Separationsstreifens ausgebildeten Öffnung bewegt wird, um das Blutserum durch die Öffnung aus dem Separationsstreifen hinaus zu drücken. Dies ist unpraktisch und birgt das Risiko der Beschädigung des Separationsstreifens und der Verschmutzung des Blutserums.

DE 20 2006 007 817 U1 beschreibt einen Separator zur Entnahme von Blutserum einer Blutprobe aus einem Separationsstreifen, mit einem Oberteil, mit einem Unterteil und mit einem Gelenk. Das Oberteil und das Unterteil sind durch das Gelenk schwenkbar miteinander verbunden und das Unterteil ist zur Aufnahme des Separationsstreifens ausgebildet. An dem Unterteil und/oder an der Unterseite des Oberteils ist wenigstens ein Vorsprung zum Ausüben von lokalem Druck auf den Separationsstreifen bei Zusammenklappen von Oberteil und Unterteil ausgebildet, so dass beim Verschieben des SeparationssVeifens Blutserum aus dem hinteren Ende des Separationsstreifens austritt.

Aufgabe der vorliegenden Erfindung ist es, eine Vorrichtung zur Verfügung zu stellen, mit der eine bestimmte Menge an Blutserum oder Blutplasma auf einfache Weise separiert werden kann, ohne den Separationsteststreifen zu beschädigen oder das Blutserum oder Blutplasma zu verschmutzen.

Diese Aufgabe wird erfindungsgemäß durch einen Separator zur Entnahme von Blutserum oder Blutplasma aus einem Separationsstreifen gemäß dem unabhängigen Anspruch 1 gelöst.

Die Erfindung betrifft demnach einen Separator zur Entnahme von Blutserum oder Blutplasma einer Blutprobe aus einem Separationsstreifen, der ein Unterteil und ein gegenüber dem Unterteil verschiebbares Oberteil aufweist. Das Unterteil kann einen Separationsstreifen aufnehmen, der hierfür auf das Unterteil oder in eine auf dem Unterteil vorgesehene Vertiefung eingelegt oder eingeschoben werden kann. An der Unterseite des Oberteils ist wenigstens ein Vorsprung vorgesehen, der lokalen Druck auf den eingelegten Separationsstreifen ausübt. Beim Verschieben des Oberteils in Richtung des vorderen Endes des eingelegten Separationsstreifens wird das Blutserum oder Blutplasma aus dem vorderen Ende herausgestreift und tritt somit aus dem Separationsstreifen aus.

Gemäß einem Grundgedanken der Erfindung wird durch einen derartigen Separator ein definierter, lokaler Druck auf den Separationsstreifen ausgeübt, durch Verschieben des Oberteils gegenüber dem Unterteil wirkt der Vorsprung auf den Separationsstreifen und somit wird das Blutserum oder Blutplasma berührungs- und beschädigungsfrei aus dem Separationsstreifen entnommen.

Durch das Anordnen und Verschieben des Oberteils in einem definierten Abstand zum Unterteil wird durch den Vorsprung ein konstanter Druck auf den Separationsstreifen ausgeübt und das Blutserum oder Blutplasma mit gleichbleibender Effizienz aus dem Separationsstreifen herausgestreift.

In einer ersten Ausführungsform dieses erfindungsgemäßen Separators ist der Vorsprung eine Rolle oder eine Walze, wodurch eine schonende Behandlung des Separationsstreifens erreicht, eine Beschädigung des Separationsstreifens vermieden und ein besonders effizientes Herausstreifen des Blutserums oder Blutplasmas gewährleistet wird. Die Rolle oder Walze ist insbesondere um eine in das Oberteil eingelassene Achse rotierbar.

Gemäß einer weiteren Ausführungsform der Erfindung kann ein elektrischer Antriebsmotor für die Rolle vorgesehen sein, der beispielsweise im Oberteil integriert ist, um das Blutserum oder Blutplasma automatisch und mit einer vorgegebenen Geschwindigkeit und somit noch zuverlässiger aus dem Separationsstreifen herauszustreifen.

Der Verschiebemechanismus des Oberteils gegenüber dem Unterteil ist erfindungsgemäß wie folgt ausgeführt: Im Unterteil sind zwei Führungsschienen vorgesehen, auf denen das Oberteil gegenüber dem Unterteil verschoben werden kann, indem zwei aus dem Oberteil herausragende laufende in die Führungsschienen eingreifende.

Um eine maximale Verschiebeposition des Oberteils gegenüber dem Unterteil zu gewährleisten, kann wenigstens ein Anschlag vorgesehen sein. Dieser kann beispielsweise durch das Ende der Führungsschiene gebildet sein, gegen die ein Gleiter oder Gleitelement anschlägt.

Gemäß einer weiteren Ausführungsform der Erfindung hat das Oberteil eine Aussparung, die so angeordnet ist, dass das aus dem hinteren Ende des Separationsstreifens ausgetretene Blutserum oder Blutplasma zugänglich ist, wenn das Oberteil gegenüber dem Unterteil verschoben worden ist und durch Einwirkung des Vorsprungs das Blutserum oder das Blutplasma aus dem hinteren Ende des Separationsstreifens herausgetreten ist. Diese Aussparung kann insbesondere in einem mittleren Bereich des hinteren Endes des Oberteils angeordnet sein.

Alternativ zu einer Aussparung kann das Oberteil kürzer ausgeführt sein, so dass dessen Ende in der maximalen Verschiebeposition so angeordnet ist, dass das aus dem hinteren Ende des Separationsstreifens ausgetretene Blutserum oder Blutplasma zugänglich ist.

Erfindungsgemäß weist die Vertiefung an beiden Längsseiten jeweils eine nasenförmige Ausformung auf.

Diese Ausformung ist so bemessen, dass sie in einen damit korrespondierenden Ausschnitt des Separationsstreifens eingreift und somit den Separationsstreifen in oder auf dem Unterteil fixiert.

In einer weiteren Ausführungsform ist eine Zeitmesseinheit mit einem Signalgeber in dem Oberteil oder dem Unterteil vorgesehen. Die Zeitmesseinheit misst ein vorgebbares Zeitintervall, und der Signalgeber gibt nach Ablauf dieses Zeitintervalls ein optisches oder akustisches Signal aus. Die Zeitmesseinheit kann über Bedientasten verfügen, mittels derer das erforderliche Zeitintervall manuell eingestellt werden kann. Der Beginn der Zeitintervallmessung kann der Zeitmesseinheit durch Betätigen einer Bedientaste, durch Einlegen des Separationsstreifenswas durch einen Schalter oder Sensor erfasst werden kann, bei der Schwenkvariante des Separators durch Zusammenklappen von Ober- und Unterteil, was durch einen Schalter oder Sensor erfasst werden kann, und bei der Schiebevariante des Separators durch Aufsetzen des Oberteils auf das Unterteil, was durch einen Schalter oder Sensor erfasst werden kann, angezeigt werden. Der Signalgeber kann in Form einer Digitalanzeige ausgebildet sein, deren Ziffern nach Ablauf des Zeitintervalls zu leuchten oder zu blinken beginnen. Der Signalgeber kann auch eine LED oder ein Summer sein.

Die Erfindung wird nachfolgend anhand von Ausführungsbeispielen mit Bezug auf die beiliegenden Figuren ausführlich beschrieben.

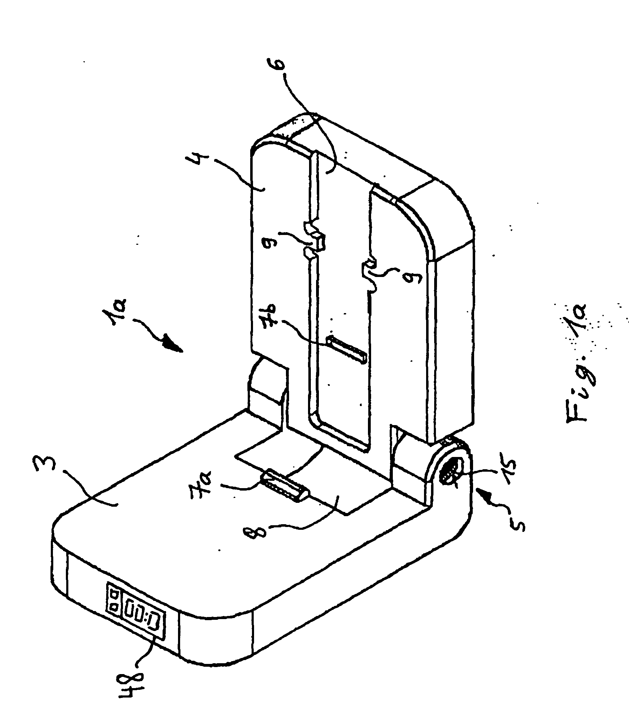

- Fig. 1a: zeigt eine perspektivische Ansicht eines bekannten Separators im aufgeklappten Zustand;

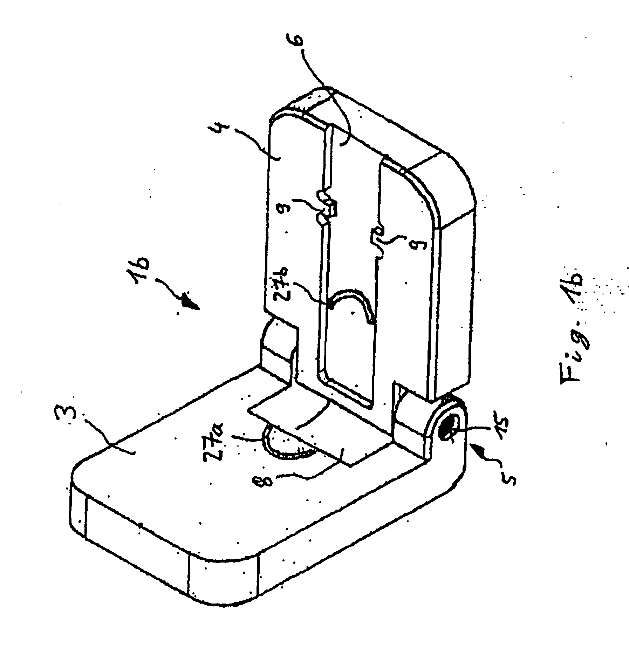

- Fig. 1b: zeigt eine perspektivische Ansicht eines bekannten Separators im aufgeklappten Zustand;

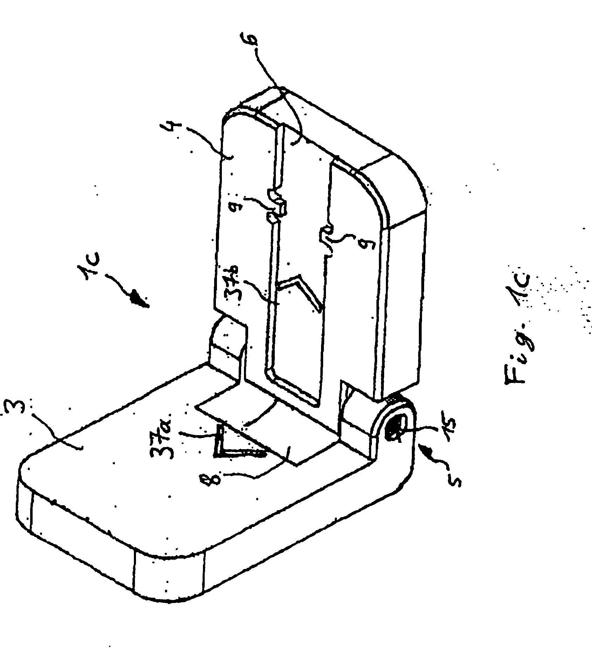

- Fig. 1c: zeigt eine perspektivische Ansicht eines bekannten Separators im aufgeklappten Zustand;

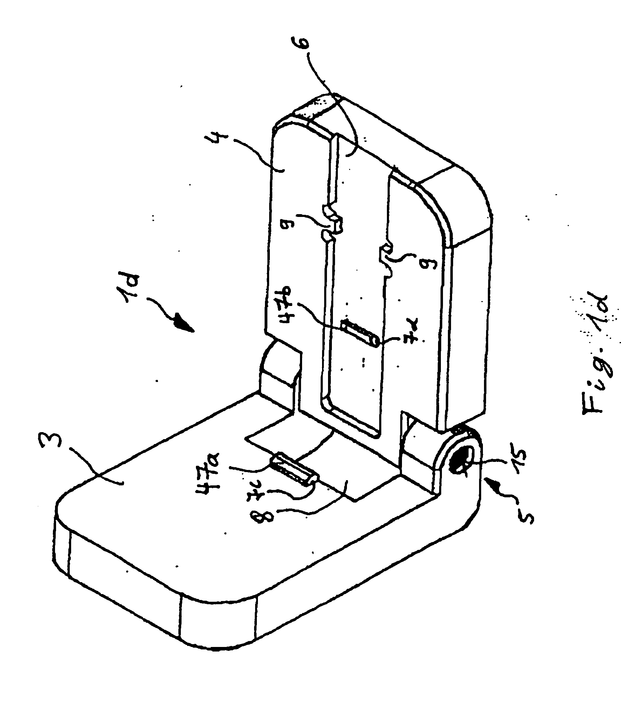

- Fig. 1d: zeigt eine perspektivische Ansicht eines bekannten Separators im aufgeklappten Zustand;

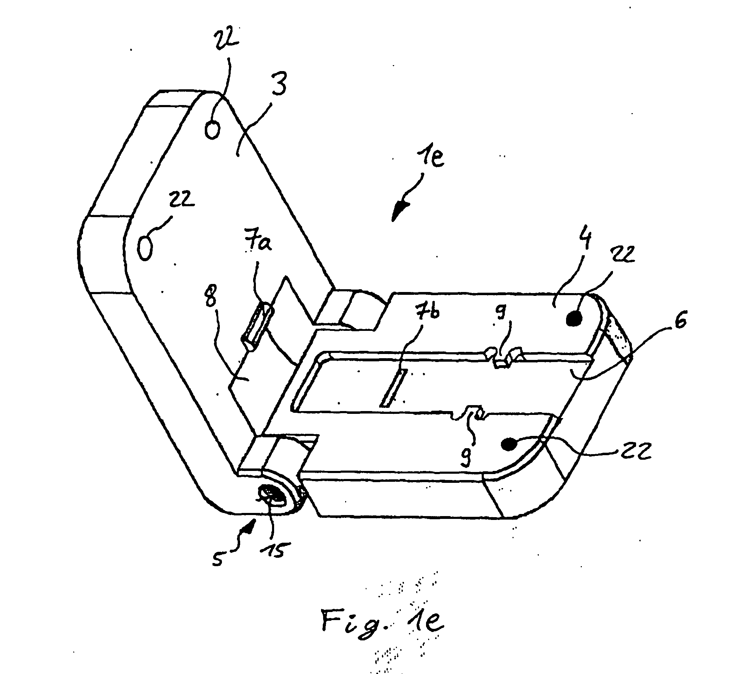

- Fig. 1e: zeigt eine perspektivische Ansicht eines bekannten Separators im aufgeklappten Zustand;
- Fig. 1: f zeigt eine perspektivische Ansicht eines bekannten Separators im aufgeklappten Zustand;
- Fig. 2a: zeigt eine Schnittdarstellung eines Separationsstreifens;
- Fig. 2b: zeigt eine Draufsicht auf den Separationsstreifen aus Fig. 2a;
- Fig. 3: zeigt eine Draufsicht auf einen zweiten Separationsstreifen;
- Fig. 4: zeigt eine Draufsicht auf einen dritten Separationsstreifen;
- Fig. 5: zeigt eine Draufsicht auf einen vierten Separationsstreifen;
- Fig. 6: zeigt eine Draufsicht auf einen fünften Separationsstreifen;
- Fig. 7: zeigt eine Draufsicht auf einen sechsten Separationsstreifen;
- Fig. 8: zeigt eine perspektivische Ansicht des Separators aus Fig. 1a im aufgeklappten Zustand, in den der Separationsstreifen aus Fig. 3 eingelegt ist;
- Fig. 9a: zeigt eine Seitenansicht des Separators aus Fig. 1a im aufgeklappten Zustand;
- Fig. 9b: zeigt eine Schnittdarstellung des Separators aus Fig. 1 a im aufgeklappten Zustand;
- Fig. 10: zeigt eine perspektivische Ansicht eines Gelenkstiftes;
- Fig. 11: zeigt eine perspektivische Ansicht eines erfindungsgemäßen Separators mit einem Unterteil und einem von diesem abgenommenen Oberteil gemäss einem Ausführungsbeispiel;
- Fig. 12: zeigt eine perspektivische Ansicht des Oberteils aus Fig. 11 schräg von unten; und
- Fig. 13: zeigt eine perspektivische Ansicht des erfindungsgemäßen Separators mit dem auf das Unterteil aufgesetzten Oberteil in einer maximalen Verschiebeposition.

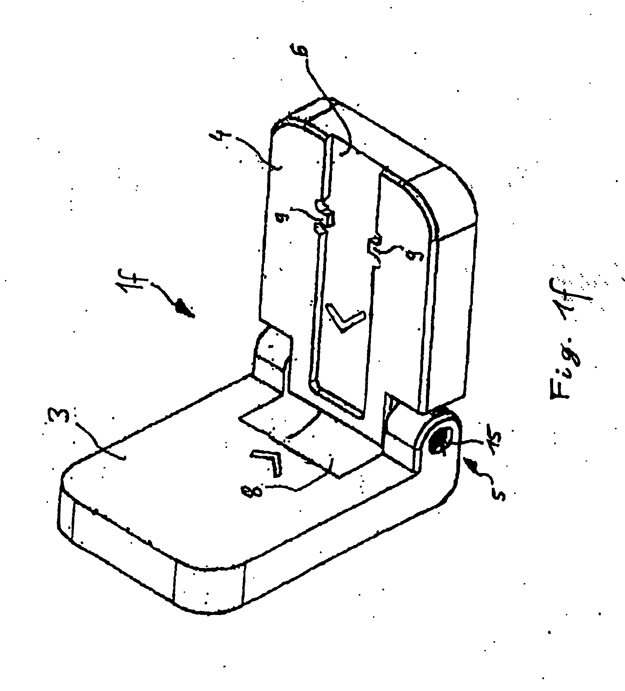

Dabei zeigen die Fig. 1 bis 10 keine Ausführungsbeispiele der beanspruchten Erfindung, sondern dienen allein der Erläuterung des technischen Hintergrunds der Erfindung.

Fig. 1a zeigt eine perspektivische Ansicht eines Separators 1a, wie er z.B. aus DE 20 2006 007 817 U1 bekannt ist, im aufgeklappten Zustand ohne einen eingelegten Separationsstreifen.

Der Separator 1a umfasst ein Unterteil 4 und ein Oberteil 3, das in einer nahezu senkrechten Position zu dem Unterteil 4 dargestellt ist. In seinem unteren Bereich weist das Oberteil 3 rechtwinklig an dem Oberteil 3 ansetzende Gelenkvorsprünge auf. Durch diese Gelenkvorsprünge und durch den von diesen Gelenkvorsprüngen eingeschlossenen, in Fig. 1a links angeordneten stufenförmigen End-abschnitt des Unterteils 4 verlaufen Bohrungen, durch die ein Gelenkstift 15 geführt ist, wodurch das Gelenk 5 gebildet wird, um das das Unterteil 4 und das Oberteil 3 geschwenkt und geklappt werden können.

Die Materialdicke und die Abmessungen des Oberteils 3 und des Unterteils 4 stimmen in etwa überein. Die sich in Fig. 1a nach rechts erstreckenden Kanten des Unterteils 4 sind abgerundet, ebenso verhält es sich mit den in Fig. 1a in oberer Richtung dargestellten Kanten des Oberteils 3. Dementsprechend fluchten die Seitenflächen des Oberteils 3 und des Unterteils 4 im zusammengeklappten Zustand in etwa miteinander.

Wie in Fig. 1a gut zu erkennen ist, weist das Oberteil 3 eine Aussparung 8 nahe dem Gelenk auf, so dass im zusammengeklappten Zustand des ersten Separators 1a dass das hintere Ende der Oberseite des Unterteils 4 von oben zugänglich ist.

Auf der Oberseite des Unterteils 4 ist eine Vertiefung 6 ausgebildet, die sich von vorne nach hinten über die Oberseite des Unterteils 4 erstreckt und nach hinten, nach links und nach rechts durch Seitenwände begrenzt und nach vorne hin offen ist. Die in Fig. 1a hinten dargestellte Seitenwand kann auch einen Durchbruch aufweisen, damit das separierte Blutserum oder Blutplasma leichter entnommen werden kann.

An der linken und der rechten Seitenwand der Vertiefung 6 sind einander gegenüberliegende, nasenförmige Ausformungen 9 ausgebildet sind, die in die Vertiefung 6 seitlich hinein ragen und somit deren Breite in diesem Bereich verringern. Die beiden nasenförmigen Ausformungen 9 sind in Fig. 1a in einem vorderen Bereich der Vertiefung 6 angeordnet, und sie stellen Anschläge 9 für einen erfindungsgemäßen Separationsstreifen dar, der nachfolgend noch näher erläutert wird.

An dem Oberteil 3, direkt benachbart der rechteckigen Aussparung 8 ist ein schwellenförmig ausgebildeter und quer zu der Vertiefung 6 verlaufender, erster Vorsprung 7a ausgebildet, der in Fig. 1a nach vorne weist und im zusammengeklappten Zustand des ersten Separators 1a demzufolge nach unten gerichtet ist.

Auf dem Boden .der Vertiefung 6 ist in einem hinteren Bereich der Vertiefung 6 ein zweiter Vorsprung 7b angeordnet, der in Fig. 1a nach oben aus der Vertiefung 6 herausragt. Dieser zweite Vorsprung 7b ist so angeordnet, dass er im zusammengeklappten Zustand des ersten Separators 1a mit dem ersten Vorsprung 7a zusammenwirkt. Der zweite Vorsprung 7b ist ebenfalls schwellenförmig und quer zur Längsrichtung der Vertiefung 6 angeordnet.

Die Vorsprünge 7a und 7b in Fig. 1a sind nur beispielhaft, sie können beliebig ausgebildet sein, bspw. kann auch einer der beiden Vorsprünge eine grössere Breite haben oder die Vorsprünge können eine gekrümmte oder gerundete Form aufweisen.

In dem Oberteil 3 ist eine Zeitmesseinheit 48 integriert, die in Fig. 1a eine Digitalanzeige und zwei rechts davon angeordnete Bedientasten umfasst. Mittels der Zeitmesseinheit 48 kann ein Zeitintervall gestoppt werden, das erforderlich ist, damit sich in dem Separationsstreifen das Blutserum oder Blutplasma durch Kapillarwirkung von den roten Blutkörperchen trennt und an dem vorderen Ende des Blutseparationsteils nahe der Blutentnahmeöffnung ansammelt. Nach Ablauf dieses Zeitintervalls kann durch den Separator 1a das Blutserum oder Blutplasma aus dem Separationsstreifen herausgestreift werden.

Mittels den Bedientasten kann das Zeitintervall manuell eingestellt werden. Beispielsweise ist für eine Blutprobe eines Erwachsenen ein Zeitintervall von etwa 0,5 Minuten, für eine Blutprobe eines Neugeborenen ein Zeitintervall von etwa 1 Minute und für eine Blutprobe eines Frühgeborenen ein Zeitintervall von etwa 2 Minuten erforderlich.

Der Zeitintervall beginnt mit Einführen einer Blutprobe in den Separationsstreifen. Der Beginn der Zeitintervallmessung wird der Zeitmesseinheit 48 durch Betätigen einer Bedientaste der Zeitmesseinheit 48 angezeigt. Alternativ dazu kann der Beginn der Zeitintervallmessung auch ab dem Zeitpunkt des Zusammenklappens von Oberteil 3 und Unterteil 4 erfolgen, was durch einen in der Figur 1a nicht gezeigten Schalter oder Sensor erfasst wird.

Nach Ablauf des Zeitintervalls kann durch die Zeitmesseinheit 48 ein akustisches oder optisches Signal ausgegeben werden, das signalisiert, dass nun der Separationsstreifen zurückgezogen werden kann, um das Blutserum oder Blutplasma herauszustreifen.

In der einfachsten Ausführungsform der Zeitmesseinheit 48 kann diese anstelle einer Digitalanzeige über eine LED, die nach Ablauf des Zeitintervalls zu leuchten oder zu blinken beginnt, und/oder über einen Summer verfügen, der nach Ablauf des Zeitintervalls ein Summton erzeugt.

Obwohl die Zeitmesseinheit 48 nur bei dem ersten Separator 1 a gezeigt ist, kann die Zeitmesseinheit 48 auch bei den folgenden Separatoren 1 b, 1 c, 1 d und 1 e integiert sein. Auf eine separate Beschreibung der Zeitmesseinheit 48 mit Bezug auf die Separatoren 1 b, 1 c, 1 d und 1 e wird verzichtet, um Wiederholungen zu vermeiden.

Fig. 1b zeigt eine perspektivische Ansicht eines zweiten Separators 1 b im aufgeklappten Zustand ohne einen eingelegten Separationsstreifen.

Der zweite Separator 1b unterscheidet sich vom ersten Separator 1 a dadurch, dass anstelle des ersten Vorsprungs 7a und des zweiten Vorsprungs 7b ein halbkreisförmig ausgebildeter dritter Vorsprung 27a und ein halbkreisförmig ausgebildeter vierter Vorsprungs 27b vorgesehen sind. Die Öffnungen des Halbkreises des dritten Vorsprungs 27a und des vierten Vorsprungs 27b zeigen in die Richtung des Gelenks 5.

Fig. 1c zeigt eine perspektivische Ansicht eines dritten Separators 1c im aufgeklappten Zustand ohne einen eingelegten Separationsstreifen.

Der dritte Separator 1 c unterscheidet sich vom ersten Separator 1 a dadurch, dass anstelle des ersten Vorsprungs 7a und des zweiten Vorsprungs 7b ein V-förmiger fünfter Vorsprung 37a und ein V-förmiger sechster Vorsprung 37b vorgesehen sind, welche die Form von Schenkeln eines gleichschenkligen Dreiecks haben, wobei die Spitze des Dreiecks, an der die beiden Schenkel miteinander verbunden sind, von dem Gelenk 5 weg zeigt.

Fig. 1d zeigt eine perspektivische Ansicht eines vierten Separators 1 d im aufgeklappten Zustand ohne einen eingelegten Separationsstreifen.

Der vierte Separator 1d unterscheidet sich von dem ersten Separator 1a, dass anstelle des ersten Vorsprungs 7a und des zweiten Vorsprungs 7b eine erste Rolle bzw. Walze 47a und eine zweite Rolle bzw. Walze 47b vorgesehen sind. Durch das Vorsehen der Rollen 47a und 47b kann der Separationsstreifen mit weniger Kraftaufwand und daher leichter bewegt und verschoben werden.

Die erste Rolle 47a und die zweite Rolle 47b sind um eine ungefähr auf Höhe der Unterseite des Oberteils 3 und der Oberseite des Unterteils 4 angeordnete und insbesondere in dem Oberteil 3 und dem Unterteil 4 angeordnete, rechtwinklig zur Längsrichtung der Vertiefung 6 bzw. zur Bewegungsrichtung des Separationsstreifens ausgerichtete erste Achse 7c sowie zweite Achse 7d drehbar gelagert, sodass sie bei einer Bewegung oder Verschiebung des Separationsstreifens in eine Drehbewegung versetzt werden.

Alternativ dazu kann wenigstens ein in Figur 1d nicht gezeigter elektrischer Antriebsmotor vorgesehen sein, der im Betrieb die Rollen 47a und 47b antreibt. Dieser Antriebsmotor ist dabei vorteilhafterweise in das Oberteil 3 oder in das Unterteil 4 des vierten Separators 1d integriert. Wenn beide Rollen 47a und 47b angetrieben werden sollen, kann ein gemeinsamer Antriebsmotor in dem Oberteil 3 oder in dem Unterteil 4 vorgesehen werden, oder es kann je ein elektrischer Antriebsmotor für die Rolle 47a in dem Oberteil 3 und für die Rolle 47b in dem Unterteil 4 vorgesehen werden.

Fig. 1e zeigt eine perspektivische Ansicht eines fünften Separators 1 e im aufgeklappten Zustand ohne einen eingelegten Separationsstreifen.

Der fünfte Separator 1 e unterscheidet sich von dem ersten Separator 1 a dadurch, dass Permanentmagnete 22 in den abgerundeten Ecken des Oberteils 3 sowie in den abgerundeten Ecken des Unterteils 4 angeordnet sind. Diese haben in der beispielhaften Darstellung gemäss Fig. 1e einen kreisförmigen Querschnitt uns sind derart angeordnet, dass sie im zusammengeklappten Zustand des fünften Separators 1e einander gegenüber liegen. Die Permanentmagnete 22 des Oberteils 3 und die Permanentmagnete 22 des Unterteils 4 sind jeweils entgegengesetzt magnetisiert, sodass sie sich gegenseitig anziehen. Dadurch wirkt beim Zusammenklappen des fünften Separators 1e eine definierte Kraft zwischen Oberteil 3 und Unterteil 4, welche ein manuelles Zusammendrücken von Oberteil 3 und Unterteil 4 ersetzt.

Fig. 1f zeigt eine perspektivische Ansicht eines sechsten Separators 1f im aufgeklappten Zustand ohne einen eingelegten Separationsstreifen.

Der sechste Separator 1f unterscheidet sich vom dritten Separator 1c dadurch, dass die V-förmigen Vorsprünge 38a und 38b die Form von Schenkeln eines gleichschenkligen Dreiecks haben, wobei die Spitze des Dreiecks, an der die beiden Schenkel miteinander verbunden sind, zu dem Gelenk 5 hin zeigt.

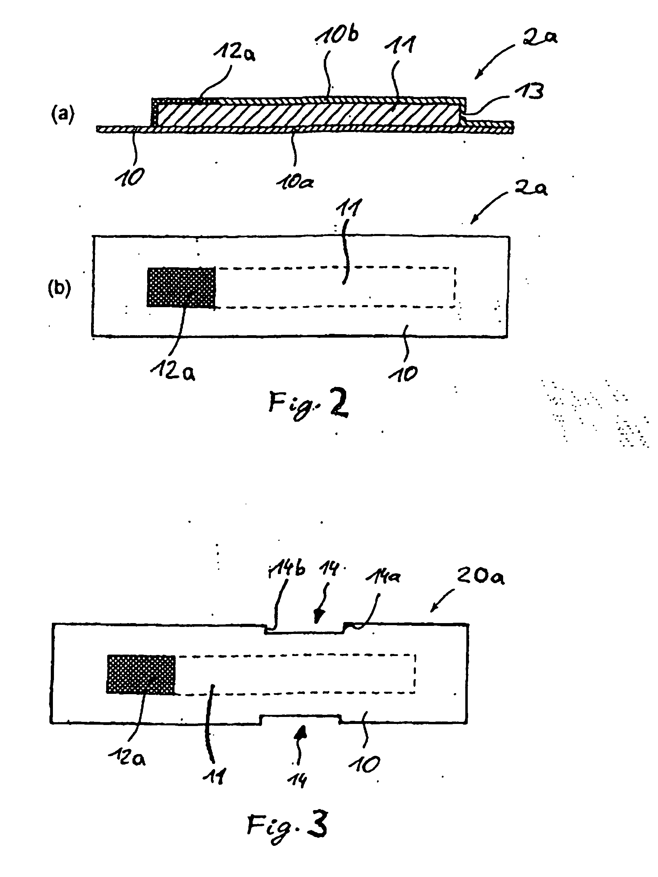

Fig. 2 zeigt einen ersten Separationsstreifen 2a in einer Schnittdarstellung (Fig. 2a) und in einer Draufsicht (Fig. 2b).

Dieser erste Separationsstreifen 2a ist zum Abscheiden von Blutserum oder Blutplasma aus einer Blutprobe bestimmt. Im mittleren Bereich der Fig. 2a ist ein Blutseparationsteil 11 dargestellt, der von einem Halteteil 10 umgeben wird. Das Halteteil 10 umfasst ein unteres Halteteil 10a und ein oberes Halteteil 10b, die das Blutseparationsteil 11 umschliessen. In dem in Fig. 2a links dargestellten vorderen Abschnitt des oberen Halteteils 10b ist ein erster Bluteinführungsabschnitt 12a ausgebildet, durch den eine Blutprobe in das Blutseparationsteil 11 eingeführt werden kann. In dem in Fig. 2a rechts dargestellten hinteren Abschnitt des oberen Halteteils 10b ist eine Blutentnahmeöffnung 13 ausgebildet.

Fig. 2b zeigt das Halteteil 10, welches das in der Draufsicht gestrichelt dargestellte Blutseparationsteil 11 umgibt. Der erste Bluteinführungsabschnitt 12a ist in dem in Fig. 2b links dargestellten vorderen Endabschnitt des Blutseparationsteils 11 gut zu erkennen.

Fig. 3 zeigt eine Draufsicht auf einen zweiten Separationsstreifen 20a.

Der zweite Separationsstreifen 20a unterscheidet sich von dem ersten Separationsstreifen 2a, wie er in den Fig. 2a und 2b dargestellt ist, dadurch, dass an den Längsseiten des zweiten Separationsstreifens 20a jeweils rechteckige Ausschnitte 14 mit einem hinteren Anschlag 14a und mit einem vorderen Anschlag 14b ausgebildet sind. Diese Anschläge 14a und 14b schlagen in dem in den Separator eingelegten Zustand jeweils an die nasenförmigen Ausformungen 9 an, so dass der zweite Separationsstreifen 20a zwischen zwei definierten Positionen bzgl. des Separators bewegt werden kann.

Der in den Fig. 2 und Fig. 3 als erster Bluteinführungsabschnitt 12a bezeichnete Bereich des Separationsstreifens 2a bzw. 20a kann auch als Separationsfeld bezeichnet werden. Die Separationsstreifen 2a und 20a sind auf ihrer Unterseite mit einer in den Fig. 2 und Fig. 3 nicht zu erkennenden laufenden Nummer versehen.

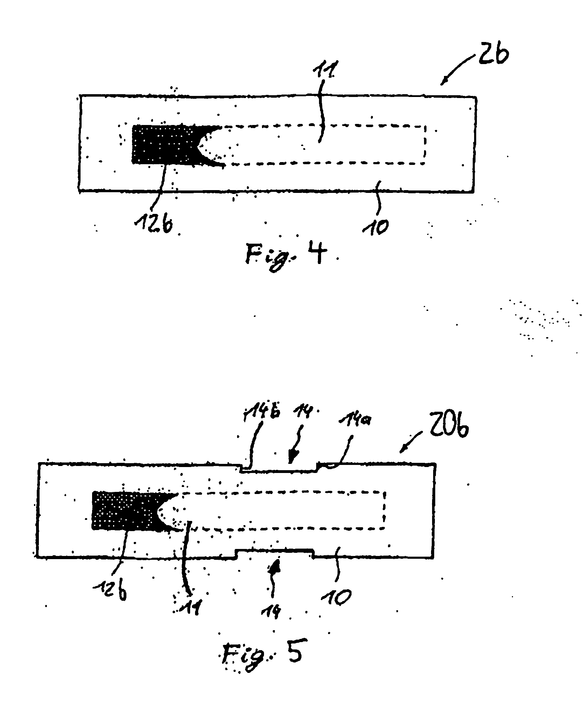

Fig. 4 zeigt eine Draufsicht auf einen dritten Separationsstreifen 2b, und Fig. 5 zeigt eine Draufsicht auf einen vierten Separationsstreifen 20b.

Der dritte Separationsstreifen 2b und der vierte Separationsstreifen 20b unterscheiden sich vom ersten Separationsstreifen 2a und vom zweiten Separationsstreifen 20a nur durch deren Bluteinführungsabschnitt 12b, dessen hintere Seite halbkreisförmig ausgebildet ist.

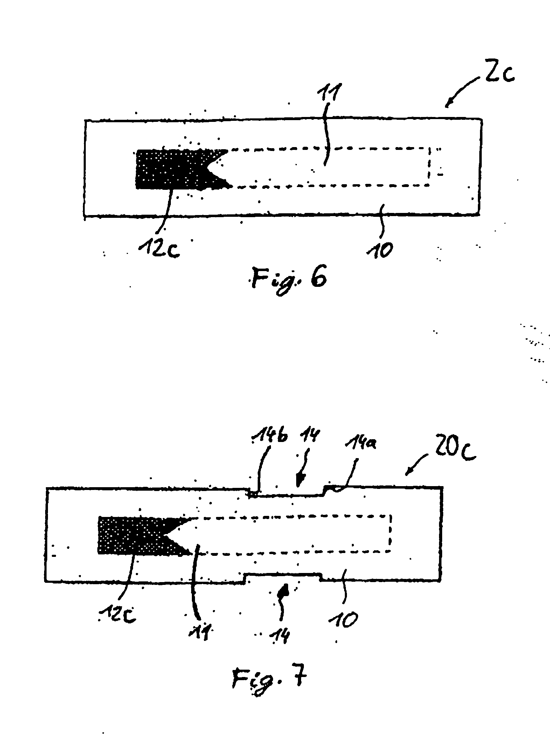

Fig. 6 zeigt eine Draufsicht auf einen fünften Separationsstreifen 2c, und Fig. 7 zeigt eine Draufsicht auf einen sechsten Separationsstreifen 20c.

Der fünfte Separationsstreifen 2c und der sechste Separationsstreifen 20c unterscheiden sich vom ersten Separationsstreifen 2a und vom zweiten Separationsstreifen 20a nur durch deren Bluteinführungsabschnitt 12c, dessen hintere Seite V-förmig ausgebildet ist.

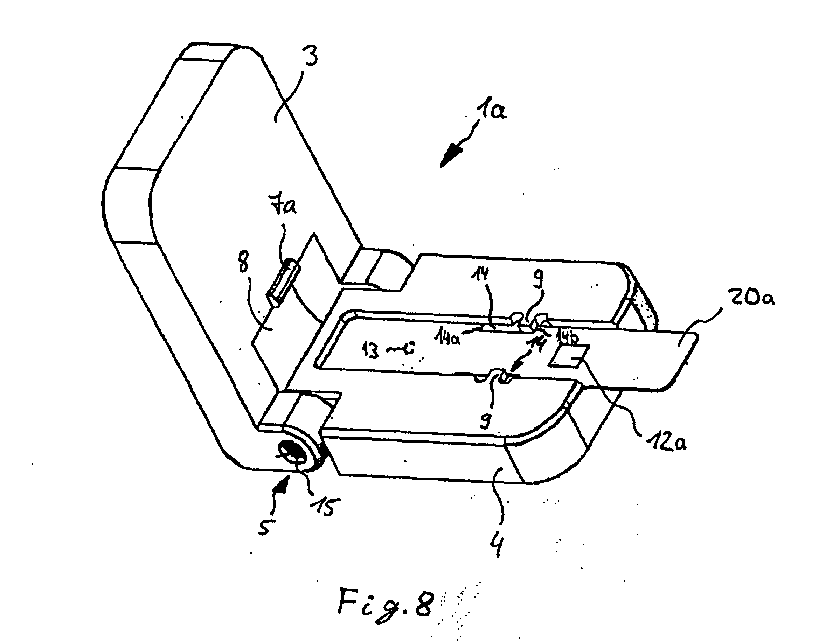

Fig. 8 zeigt eine perspektivische Ansicht des ersten Separators 1a im aufgeklappten Zustand mit eingelegtem zweiten Separationsstreifen 20a.

Dabei ist an der Oberseite des zweiten Separationsstreifens 20a der erste Bluteinführungsabschnitt 12a gut zu erkennen. Die ebenfalls an der Oberseite des zweiten Separationsstreifens 20a angeordnete Blutentnahmeöffnung 13 ist an dem in Fig. 8 links dargestellten hinteren Ende des zweiten Separationsstreifens 20a gestrichelt dargestellt. Der in der Vertiefung 6 ausgebildete zweite Vorsprung 7b ist in Fig. 4 demgemäss nicht zu erkennen, zumal er von dem zweiten Separationsstreifen 20a verdeckt wird.

In Fig. 8 ist ebenfalls gut zu erkennen, dass die vorderen Anschläge 14b der Ausschnitte 14 des zweiten Separationsstreifens 20a an den nasenförmigen Ausformungen 9 anliegen, und dass sich der zweite Separationsstreifen 20a somit in der eingeschobenen Position befindet.

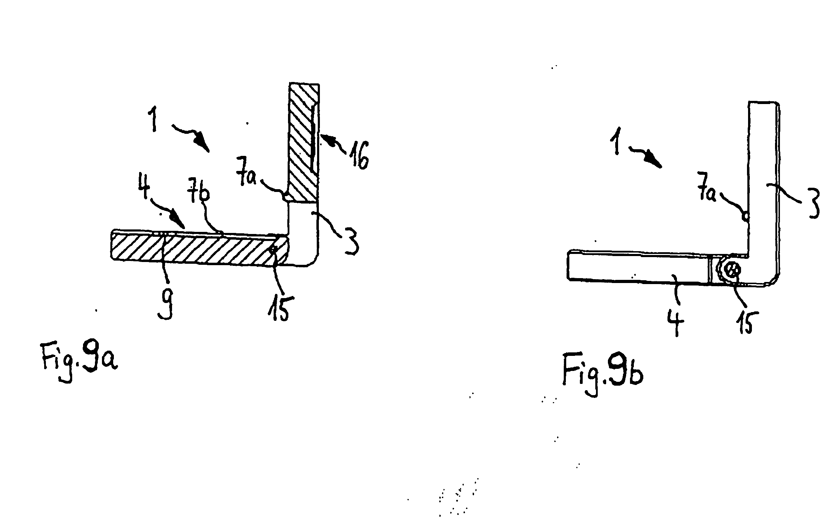

Fig. 9a zeigt eine Darstellung eines Längsschnitts des ersten Separators 1 a im aufgeklappten Zustand.

Im oberen Teil ist das senkrecht angeordnete Oberteil 3 zu erkennen, das durch den Gelenkstift 15 schwenkbar mit dem in Fig. 9a waagerecht ausgerichteten Unterteil 4 verbunden ist. An der nach hinten weisenden Oberseite des Oberteils 3 ist eine Ausnehmung 16 angeordnet, in die bspw. eine Typenbezeichnung oder ein Firmenlogo eingebracht ist. An der Unterseite des Oberteils 3 ist der erste Vorsprung 7a zu erkennen, der nach vorne weist. An der Oberseite des Unterteils 4 sind die an den Seitenwänden der Vertiefung ausgebildeten nasenförmigen Ausformung 9 ebenso zu erkennen, wie der am Boden der Vertiefung 6 ausgebildete zweite Vorsprung 7b.

Fig. 9b zeigt eine Seitenansicht des ersten Separators 1 a im aufgeklappten Zustand.

Dabei sind das waagrecht angeordnete Unterteil 4 und das dazu senkrecht angeordnete Oberteil 3 zu erkennen. Des weiteren ist der an der Unterseite des Oberteils 3 angeordnete erste Vorsprung 7a zu erkennen.

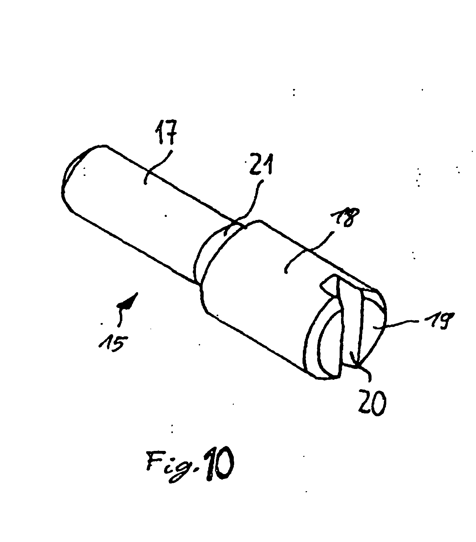

Fig. 10 zeigt eine perspektivische Ansicht des Gelenkstifts 15.

Dabei umfasst der Gelenkstift 15 einen Kopf 18 mit einer Stirnfläche 19, in die ein Schlitz 20 eingebracht ist, in den ein Schraubenzieher eingreifen und so den Gelenkstift 15 bewegen oder drehen kann. Des weiteren verfügt der Gelenkstift 15 über einen Bolzen 17 der einen geringeren Durchmesser aufweist als der Kopf 18 und der von dem Kopf 18 durch eine Einkerbung 21 beabstandet ist. In Fig. 10 ist der Bolzen 17 verkürzt dargestellt. In der Praxis ist der Bolzen so lang ausgebildet, dass er sich von dem Kopf 18 durch das Unterteil 4 bis zu dem gegenüberliegenden Gelenkabschnitt des Oberteils 3 erstreckt.

Der Separator 1a, 1b, 1c, 1d, 1e und 1f mit dem zweiten Separationsstreifen 20a wird wie folgt bedient:

Zunächst wird eine Blutprobe von etwa 50 µl Blut bspw. mit einer Pipette auf den ersten Bluteinführungsabschnitt 12a gegeben. Dabei wird durch Kapillarwirkung das Blutserum oder Blutplasma von den roten Blutkörperchen so getrennt, dass sich das Blutserum oder Blutplasma an dem hinteren Ende des Blutseparationsteils 11 nahe der Blutentnahmeöffnung 13 ansammelt.

Danach wird der zweite Separationsstreifen 20a, wie in Fig. 8 dargestellt, mit nach oben weisendem ersten Bluteinführungsabschnitt 12a so in den Separator 1a, 1 b, 1c, 1d, 1e und 1f gelegt, dass die vorderen Anschläge 14b an den nasenförmigen Ausformungen 9 anschlagen. Der zweite Separationsstreifen 20a befindet sich somit im eingeschobenen Zustand.

Nach Ablauf eines Zeitintervalls von etwa 0,5 bis 2 Minuten, je nach Analyt, werden das Oberteil 3 und das Unterteil 4 zusammengeklappt, und der Separator wird somit geschlossen. Das Oberteil 3 und das Unterteil 4 werden leicht aufeinandergedrückt gehalten und gleichzeitig wird der zweite Separationsstreifen 20a vorsichtig zurückgezogen, bis die hinteren Anschläge 14a an die nasenförmigen Ausformungen 9 anschlagen. Bei Verwendung des fünften Separators 1 e mit Permanentmagneten 22 kann das manuelle Aufeinanderdrücken von Oberteil 3 und Unterteil 4 entfallen, da Oberteil 3 und Unterteil 4 durch magnetische Kraftwirkung gegeneinander gedrückt werden.

Durch die Einwirkung der Vorsprünge 7a und 7b auf das Blutseparationsteil 11 wird das Blutserum oder Blutplasma nun aus der Blutentnahmeöffnung 13 herausgestreift und tritt an der Spitze des zweiten Separationsstreifens 20a aus.

Bei Verwendung des zweiten Separators 1b, des dritten Separators 1c oder des sechsten Separators 1f wird durch die halbkreisförmigen Vorsprünge 27a und 27b bzw. durch die V-förmigen Vorsprünge 37a und 37b bzw. 38a und 38b beim Herausziehen des zweiten Separationsstreifens 20a aus der Vertiefung 6 das Blutserum oder Blutplasma in der Mitte des zweiten Separationsstreifens 20a konzentriert und mittig aus dem zweiten Separationsstreifen 20a herausgedrückt.

Bei Verwendung des vierten Separators 1d wird durch die Rollen 47a und 47b beim Herausziehen des zweiten Separationsstreifens 20a aus der Vertiefung 6 das Blutserum oder Blutplasma besonders schonend aus dem zweiten Separationsstreifen 20a herausgedrückt.

Wird anstelle des zweiten Separationsstreifens 20a ein vierter Separationsstreifen 20b mit einem halbkreisförmigen Bluteinführungsabschnitt 12b oder ein sechster Separationsstreifen 20c mit einem V-förmigen Bluteinführungsabschnitt 12c verwendet, so wird das Blutserum oder Blutplasma noch besser in der Mitte des Blufeinführungsabschnitt 12b und 12c konzentriert und herausgedrückt.

Das Blutserum oder Blutplasma kann nun berührungsfrei entnommen werden, bspw. kann es mit einer Kapillarküvette aufgesaugt werden. Schliesslich ist die Kapillarküvette insbesondere mit einem Verschlusskit zu versiegeln, um das Ausfliessen des Blutserums oder des Blutplasmas zu verhindern. Die Kapillarküvette ist nun zur Messung bereit.

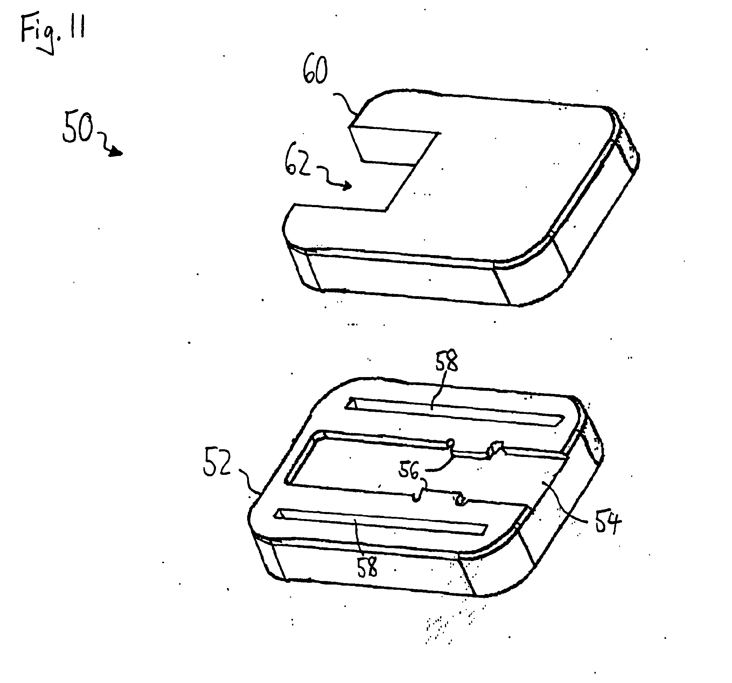

Fig. 11 zeigt eine perspektivische Ansicht eines erfindungsgemäßen Separators 50 mit einem Unterteil 52 und einem von diesem abgenommenen Oberteil 60.

Das Unterteil 52 und das Oberteil 60 haben jeweils die gleiche Dicke und eine jeweils im Wesentlichen rechteckige Form mit abgerundeten Ecken, wobei im mittleren Bereich des hinteren Endes des Oberteils 60 eine rechteckige Aussparung 62 angeordnet ist. In den Seitenbereichen der Oberfläche des Unterteils 52 sind zwei längliche Führungsschienen 58 ausgebildet, die in Fig. 11 in Form von Längsnuten ausgebildet sind. Auf der Oberseite des Unterteils 52 ist eine längliche Vertiefung 54 ausgebildet, die sich von vorne nach hinten über die Oberseite des Unterteils 52 erstreckt und die nach hinten, nach links und nach rechts durch Seitenwände begrenzt und nach vorne hin offen ist. Die hintere Seitenwand kann dabei auch einen Durchbruch aufweisen, damit das separierte Blutserum oder Blutplasma leichter entnommen werden kann.

An der linken und der rechten Seitenwand der Vertiefung 54 sind in einem vorderen Bereich einander gegenüberliegende Fixierungsnasen 56 angeordnet, deren Breite jeweils der Breite der Ausschnitte 14 der Separationsstreifen 20a, 20b und 20c entspricht. Durch diese Fixierungsnasen 56 kann ein in die Vertiefung 54 eingelegter Separationsstreifen 20a, 20b, 20c zuverlässig fixiert und in Position gehalten werden.

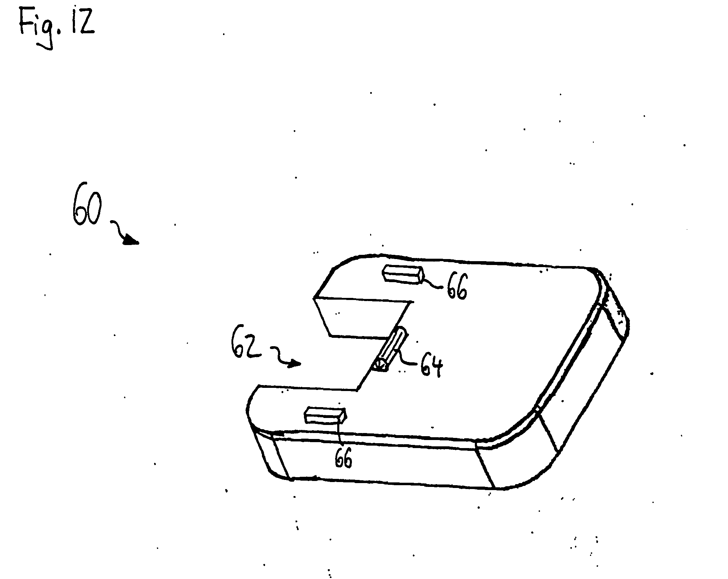

Fig. 12 zeigt eine perspektivische Ansicht des Oberteils 60 schräg von unten.

Direkt vor der rechteckigen Aussparung 62 an der Hinterseite des Oberteils 60 ist eine Rolle 64 angeordnet, die um eine ungefähr auf der Höhe der Unterseite des Oberteils 60 angeordnete sowie rechtwinklig zur Längsrichtung der Vertiefung 54 bzw. zur Bewegungsrichtung des Separationsstreifens ausgerichtete Achse drehbar gelagert ist. Die Rolle 64 steht somit ein Stück weit nach unten aus der Unterseite des Oberteils 60 heraus, so dass die Rolle 64 bei einer Verschiebebewegung des Separationsstreifens abrollt und unter Druckeinwirkung auf den Separationsstreifen das Blutserum oder Blutplasma aus diesem herausstreift.

An den Seitenbereichen des Oberteils 60 neben der Aussparung 62 ragen zwei Gleiter 66 nach unten aus dessen Oberfläche heraus. Diese Gleiter 66 können in die Führungsschienen 58 des Unterteils 52 eingesetzt werden und führen das Oberteil 60 während seiner Schiebebewegung in den Führungsschienen 58. Die Gleiter 66 sind in Bezug auf die Längsrichtung des sechsten Separators 50 hinter der Rolle 64 angeordnet, so dass sie an die hinteren Enden der Führungsschienen 58 anschlagen und die Verschiebebewegung des Oberteils 60 nach hinten begrenzen, so dass die Rolle 64 das Blutserum oder Blutplasma aus der Blutentnahmeöffnung 13 zwar herausstreift, aber nicht mit dem herausgestreiften Blutserum oder Blutplasma im hinteren Abschnitt des Separationsstreifens 20a, 20b, 20c in Berührung kommt.

Durch die oberteitseitigen Gleiter 66 und die unterteilseitigen Führungsschienen 58 wird somit ein Verschiebemechanismus gebildet, mit dem das Oberteil 60 gegenüber dem Unterteil 52 verschoben werden kann.

Dieser Verschiebemechanismus ist in den Figuren 11 bis 13 nur beispielhaft angegeben, er kann innerhalb des von Anspruch 1 vorgegebenen Rahmens auf beliebige Weise ausgeführt werden. Beispielsweise können Führungsschienen in den Seitenflächen des Unterteils 52 vorgesehen sein, und das Oberteil 60 kann breiter als das Unterteil 52 ausgebildet sein und Gleiter aufweisen, die in die seitlichen Führungsschienen des Unterteils 52 eingreifen. Dabei ist das Oberteil 60 gegenüber dem Unterteil 52 so weit nach hinten verschiebbar, dass die Vertiefung 54 freiliegt und der Separationsstreifen 20a, 20b, 20c eingelegt werden kann. Wie bei der Ausführung gemäß den Figuren 11 bis 13 können die Gleiter in Bezug auf die Längsrichtung so angeordnet sein, so dass sie an die hinteren Enden der seitlichen Führungsschienen anschlagen und die Verschiebebewegung des Oberteils 60 nach hinten begrenzen, so dass die Rolle 64 das Blutserum oder Blutplasma aus der Blutentnahmeöffnung 13 zwar herausstreift, aber nicht mit dem herausgestreiften Blutserum oder Blutplasma im hinteren Abschnitt des Separationsstreifens 20a, 20b, 20c in Berührung kommt.

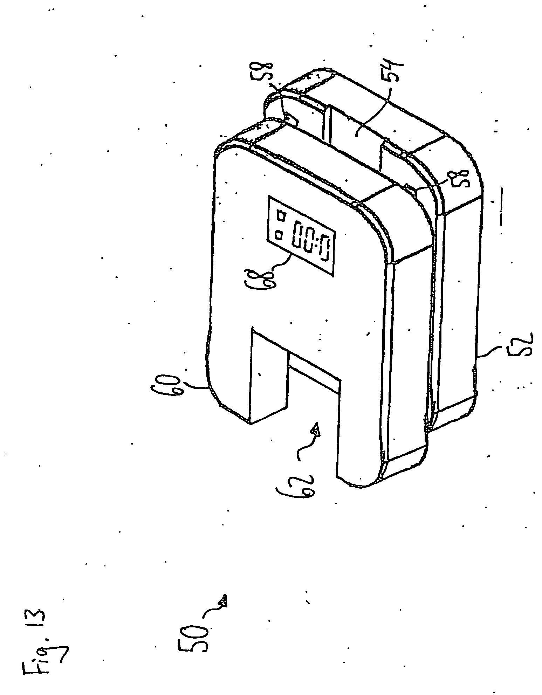

Fig. 13 zeigt eine perspektivische Ansicht des erfindungsgemäßen Separators 50 mit dem auf das Unterteil 52 aufgesetzten Oberteil 60 in einer maximalen Verschiebeposition.

Dabei sind, was in Fig. 13 nicht zu sehen ist, die Gleiter 66 an die hinteren Enden der Führungsschienen 58 angeschlagen, und das hintere Ende des Oberteils 60 steht über dasjenige des Unterteils 52 heraus.

Des weiteren ist auf der Oberseite des Oberteils 60 in einem vorderen Bereich eine Zeitmesseinheit 68 mit einer Digitalanzeige und mit zwei Bedientasten angeordnet, die der Zeitmesseinheit 48 des ersten Separators 1a entspricht.

Der erfindungsgemäße Separator 50 mit dem Separationsstreifen 20a, 20b, 20c wird wie folgt bedient:
Zunächst wird der Separationsstreifen 20a, 20b, 20c mit nach oben weisendem ersten Bluteinführungsabschnitt 12a so in den Separator 50 gelegt, dass die Fixierungsnasen 56 in den Ausschnitt 14 eingreifen und dass der Separationsstreifen 20a, 20b, 20c so gegenüber dem Unterteil 52 fixiert und in Position gehalten wird. Dann wird eine Blutprobe von etwa 50 µl Blut beispielsweise mit einer Pipette auf den ersten Bluteinführungsabschnitt 12a, 12b, 12c gegeben, und das Blutserum bzw. das Blutplasma trennt sich durch Kapillarwirkung von den roten Blutkörperchen derart, dass es sich an dem hinteren Ende des Blutseparationsteils 11 nahe der Blutentnahmeöffnung 13 ansammelt. Nun wird das Oberteil 60 fluchtend auf das Unterteil 52 aufgesetzt, so dass die Gleiter 66 in den Führungsschienen 58 angeordnet sind. Entweder durch Tastendruck oder durch einen Schalter oder Sensor, der das Aufsetzen des Oberteils 60 auf das Unterteil 52 erkennt, wird die Zeitmesseinheit 68 gestartet. Nach Ablauf des vorgegebenen Zeitintervalls gibt dieser ein akustisches oder optisches Signal aus. Danach wird das Oberteil 60 manuell oder motorisch über einen integrierten Elektromotor gegenüber dem Unterteil 52 nach hinten verschoben, bis die Gleiter 66 gegen die Enden der Führungsschienen 58 anschlagen. Dabei wird durch das Einwirken der Rolle 64 auf das Blutseparationsteil 11 das Blutserum bzw. das Blutplasma aus der Blutentnahmeöffnung 13 herausgestreift und tritt an der Spitze des Separationsstreifens 20a, 20b, 20c aus.

Das Blutserum oder das Blutplasma kann nun durch die Aussparung 62 des Oberteils 60 entnommen werden, beispielsweise kann es mit einer Kapillarküvette aufgesaugt werden. Schließlich ist die Kapillarküvette insbesondere mit einem Verschlusskit zu versiegeln, um das Ausfließen des Blutserums zu verhindern. Die Kapillarküvette ist nun zur Messung bereit.

## Patentansprüche

1. Separator (50) zur Entnahme von Blutserum oder Blutplasma einer Blutprobe aus einem Separationsstreifen (2a-c; 20a-c),
mit einem Unterteil (52) und mit einem gegenüber dem Unterteil (52) verschiebbaren Oberteil (60).
wobei an der Oberseite des Unterteils (52) eine längliche Vertiefung (54) zur Aufnahme des Separationsstreifens (2a-c; 20a-c) ausgebildet ist, die an zwei gegenüberliegenden Seiten durch Seitenwände begrenzt ist, wobei an jeder der gegenüberliegenden Seitenwände jeweils eine nasenförmige Ausformung (56) zum Fixieren des in die Vertiefung (54) eingelegten Separationsstreifens ausgebildet ist, die seitlich in die Vertiefung hineinragt.
wobei an der Unterseite des Oberteils (60) wenigstens ein Vorsprung (64) zum Ausüben eines definierten, lokalen Drucks auf den Separationsstreifen (2a-c; 20a-c) vorgesehen ist, wobei der Vorsprung (64) derart ausgebildet ist, dass beim Verschieben des Oberteils (60) gegenüber dem Unterteil (52) Blutserum oder Blutplasma aus dem hinteren Ende des Separationsstreifens (2a-c; 20a-c) austritt,
wobei aus dem Oberteil (60) zwei Gleiter (66) herausragen und in dem Unterteil (52) zwei Führungsschienen (58) zur Aufnahme der Gleiter (66) ausgebildet sind, um das Oberteil während der Schiebebewegung zu führen.

2. Separator (50) nach Anspruch 1,
**dadurch gekennzeichnet, dass**
der Vorsprung eine Rolle (64) ist

3. Separator (50) nach Anspruch 2,
**dadurch gekennzeichnet, dass**
ein Antriebsmotor für die Rolle (64) vorgesehen ist.

4. Separator (50) nach einem der Ansprüche 1 bis 3,
**dadurch gekennzeichnet, dass**
das Oberteil (60) eine Aussparung (62) hat, die so angeordnet ist, dass das aus dem hinteren Ende des Separationsstreifens (2a-c; 20a-c) ausgetretene Blutserum oder Blutplasma zugänglich ist.

5. Separator (1a-f; 50) nach einem der Anspruche 1 bis 4,
**dadurch gekennzeichnet, dass**
eine Zeitmesseinheit (48) in dem Oberteil (60) oder dem Unterteil (52) vorgesehen ist, die ein vorgebbares Zeitintervall misst und nach Ablauf dieses Zeitintervalls ein optisches oder akustisches Signal ausgibt**.**

## Claims

1. Separator (50) for the taking of blood serum or blood plasma contained in a blood sample from a separating strip (2a-c; 20a-c),
comprising a bottom part (52) and a top part (60), which is slidable with respect to the bottom part (42);
wherein an elongated indentation (54) for receiving the separating strip (2a-c; 20a-c) is formed on the upper side of the bottom part (52), the indentation (54) being limited by side walls on two opposite sides, wherein at each of the opposite side walls a nose-shaped lug (56) laterally protruding into the indentation is formed for fixing the separating strip inserted into the indentation (54),
wherein at least one protrusion (64) for exerting a defined local pressure on the separating strip (2a-c; 20a-c) is provided at the lower side of the top part (60), wherein the protrusion (64) is formed so that blood serum or blood plasma comes out of the rear end of the separating strip (2a-c; 20a-c) when the top part (60) is slid with respect to the bottom part (52),
wherein two sliders (66) protrude from the top part (60) and two guiding rails (58) for receiving the sliders (66) are formed in the bottom part (52) in order to guide the top part during the sliding movement.

2. Separator (50) according to Claim 1,
**characterized in that**
the protrusion is a roll (64).

3. Separator (50) according to Claim 1,
**characterized in that**
a driving motor for the roll (64) is provided.

4. Separator (50) according to any of Claims 1 to 3,
**characterized in that**
the top part (60) has a cut-out (62) which is arranged so that the blood serum or blood plasma, which has come out from the rear end of the separating strip (2a-c; 20a-c), is accessible.

5. Separator (50) according to any of Claims 1 to 4,
**characterized in that**
a time measurement unit (48), which measures a time interval which can be predetermined and emits an optical or acoustical signal after the expiration of said time interval, is provided in the top part (60) or in the bottom part (52).

## Revendications

1. Séparateur (50) pour prélever du sérum sanguin ou du plasma sanguin d'un échantillon de sang à partir d'une bande de séparation (2a-c ; 20a-c) ;
comprenant une partie inférieure (52) et une partie supérieure (60) pouvant être déplacée en face de la partie inférieure (52),
dans lequel il est formé, sur la face supérieure de la partie inférieure (52), un creux longitudinal (54) destiné à recevoir la bande de séparation (2a-c ; 20a-c), qui est délimité sur deux faces opposées par des parois latérales, et dans lequel il est formé, respectivement sur chacune des parois latérales opposées, un façonnage (56) en forme de nez destiné à fixer la bande de séparation placée dans le creux (54) et s'enfonçant latéralement dans le creux,
dans lequel il est prévu, sur la face inférieure de la partie supérieure (60), au moins une saillie (64) pour exercer une pression locale définie sur la bande de séparation (2a-c ; 20a-c), dans lequel la saillie (64) est formée de telle sorte que, lors du déplacement de la partie supérieure (60) face à la partie inférieure (52), du sérum sanguin ou du plasma sanguin puisse sortir de l'extrémité arrière de la bande de séparation (2a-c) ; 20a-c), et
dans lequel deux coulisseaux (66) font saillie à partir de la partie supérieure (60), et deux rails de guidage (58) destinés à recevoir les coulisseaux (66) sont formés dans la partie inférieure (52), afin de guider la partie supérieure lors du glissement.

2. Séparateur (50) selon la revendication 1,
**caractérisé en ce que**
la saillie est un rouleau (64).

3. Séparateur (50) selon la revendication 2,
**caractérisé en ce que**
l'on prévoit un moteur d'entraînement pour le rouleau (64).

4. Séparateur (50) selon l'une quelconque des revendications 1 à 3,
**caractérisé en ce que**
la partie supérieure (60) présente un évidement (62), lequel est agencé de telle sorte que le sérum sanguin ou le plasma sanguin sortant de l'extrémité arrière de la bande de séparation (2a-c ; 20a-c) soit accessible.

5. Séparateur (50) selon l'une quelconque des revendications 1 à 4,
**caractérisé en ce que**
l'on prévoit une unité de mesure du temps (48) dans la partie supérieure (60) ou la partie inférieure (52), laquelle mesure un intervalle de temps pouvant être prédéfini et produit un signal optique ou acoustique à l'expiration dudit intervalle de temps.
